(19) Europäisches Patentamt / European Patent Office / Office européen des brevets

(11) EP 4 331 500 A1

(12) **EUROPEAN PATENT APPLICATION**

(43) Date of publication:
06.03.2024 Bulletin 2024/10

(21) Application number: 22198936.1

(22) Date of filing: 30.09.2022

(51) International Patent Classification (IPC):
**A61B 8/08** (2006.01)    **A61B 8/02** (2006.01)
**A61B 8/00** (2006.01)

(52) Cooperative Patent Classification (CPC):
**A61B 8/0883; A61B 8/02; A61B 8/488;**
**A61B 8/5223;** A61B 8/463; A61B 8/469

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: 31.08.2022 PCT/CN2022/116007

(71) Applicant: **Koninklijke Philips N.V.**
**5656 AG Eindhoven (NL)**

(72) Inventors:
• **XU, Jingping**
**Eindhoven (NL)**
• **XIE, Hua**
**Eindhoven (NL)**
• **CHEN, Jin Ning**
**5656AG Eindhoven (NL)**
• **ERRICO, Claudia**
**5656AG Eindhoven (NL)**
• **SHIH, Cho-chiang**
**Eindhoven (NL)**
• **TONG, Ling**
**Eindhoven (NL)**
• **WEI, Vivian**
**Eindhoven (NL)**

(74) Representative: **Philips Intellectual Property & Standards**
**High Tech Campus 52**
**5656 AG Eindhoven (NL)**

(54) **MEASUREMENTS FROM PULSE WAVE DOPPLER ULTRASOUND**

(57)    An ultrasound imaging system (100) may automatically analyze a pulse wave Doppler spectrogram to determine high quality and low quality cardiac cycles. Based on the determination, the ultrasound imaging system (100) may exclude the low quality cardiac cycle from being used in calculations of parameter measurements such as peak systolic velocity, end diastolic velocity, and resistance index. In some examples, the ultrasound imaging system (100) may provide a visual indication on the display (138) which cardiac cycles were excluded from the measurements.

FIG. 1

**Description**

FIELD OF THE INVENTION

This application relates to pulse wave Doppler ultrasound. More specifically, this application relates to improving accuracy of measurements derived from pulse wave Doppler signals.

BACKGROUND OF THE INVENTION

Strokes are the leading cause of disability and second leading cause of death worldwide. There are two main types of strokes: (1) hemorrhagic due to bleeding (e.g. from head trauma), and (2) ischemic (i.e., due to a lack of blood flow) which accounts for approximately 87% of all stroke cases. China especially faces high death rates due to cerebrovascular diseases accounting for 1.57 million deaths in 2018. Ultrasound imaging for carotid artery assessment is widely accepted in clinical practice as first-line screening modality for stroke imaging.

Doppler ultrasound is an important step in a carotid ultrasound exam protocol. It provides information about the hemodynamics of the carotid blood flow and the severity of stenosis, which are indicators of the risk of ischemia. Doppler parameters such as Resistance Index (RI), Peak Systolic Velocity (PSV) and End Diastolic Velocity (EDV), derived from Pulse Wave (PW) Doppler spectra, have been accepted as diagnostic metrics for evaluating carotid hemodynamic and stenosis for patients at high risk of developing a stroke. RI is calculated as:

$$RI = \frac{PSV - EDV}{PSV} \qquad \text{Equation 1}$$

In clinical practice, clinicians prefer to estimate the RI by averaging the measurements over several cardiac cycles, typically using 3-5 cycles. During PW acquisition, clinicians must keep the probe still and patients must minimize movement, in order to get a good Doppler spectrum. However, sometimes the acquired spectrum still contains low-quality cardiac cycles caused by the motion from the clinician or the patient. In addition, the subject under carotid imaging may have cardiac comorbidity with irregular heart beating which will also affect the PW measurements. Irregular heart beat is determined from ultrasound Doppler data without using conventional ECG (electrocardiography) signal. This is because ECG is usually not available in regular general imaging for stroke screening, whereas ECG is typically available in echocardiographic examination. If the clinician sets the duration of 3~5 cardiac cycles spectrum for key Doppler measurements without quality check, the traditional auto-PW measurement tool could include low quality cycles which will result in inaccurate measurement. In some cases, experienced clinicians must correct them manually.

A typical carotid ultrasound examination protocol usually lasts around 10-20 minutes for both right and left carotid artery including common carotid artery (CCA), external carotid artery (ECA) and internal carotid artery (ICA), imaged using a one-dimensional (1D) probe sweep along the transverse plane or/and longitudinal plane. So there are a lot of efforts needed from the sonographer or ultrasound doctors for this highly repetitive routine work where novel automatic solution is strongly needed to reduce user's workload and simultaneously improve measurement accuracy.

SUMMARY OF THE INVENTION

Apparatuses, systems, and methods disclosed herein may automatically exclude spectral Doppler data corresponding to cardiac cycles associated with high heart rate variability and/or low acquisition quality. The excluding may include identifying and/or removing the spectral Doppler data from further parameter measurements. This may lead to more reliable parameter measurements that are based on the spectral Doppler data.

The invention is defined by the independent claims. Dependent claims define advantageous embodiments.

In accordance with at least one example disclosed herein, a system may include a non-transitory computer readable memory storing spectral Doppler data, a display, at least one processor configured to determine whether individual ones of a plurality of cardiac cycles in the spectral Doppler data are of high quality or of low quality, based, at least in part on heart rate variability, acquisition quality, or a combination thereof, and generate display data based on the determination, wherein the display is configured to provide a visual indication of one or more of cardiac cycles of the plurality of cardiac cycles determined to be of high quality and cardiac cycles of the plurality of cardiac cycles determined to be of low quality based on the display data.

In some examples, the display is configured to provide the spectral Doppler data as a spectrogram. In some examples, the visual indication comprises highlighting the cardiac cycles of the plurality of cardiac cycles determined to be of high quality in a first color and highlighting the cardiac cycles of the plurality of cardiac cycles determined to be of low quality in a second color different than the first color. In some examples, the visual indication comprises displaying a portion of the spectrogram associated with the cardiac cycles of the plurality of cardiac cycles determined to be of high quality in a different color, a different line weight, a different line texture, or a combination thereof than the cardiac cycles of the plurality of cardiac cycles determined to be of low quality.

In some examples, at least one processor is further configured to determine a parameter measurement based on cardiac cycles of the plurality of cardiac cycles determined to be of high quality. In some examples, the display is further configured to provide the parameter measurement.

In some examples, the system may further include a buffer configured to store additional Doppler spectral data, wherein when all of the plurality of cardiac cycles are determined to be of low quality, the at least one processor is further configured to determine whether individual ones of a plurality of cardiac cycles in the additional spectral Doppler data are of high quality or of low quality, based, at least in part on the heart rate variability, the acquisition quality, or the combination thereof. In some examples, when the buffer does not include additional spectral Doppler data, the at least one processor is configured to cause the display to prompt a user to acquire the additional spectral Doppler data.

In some examples, the system may further include an ultrasound probe configured to acquire the spectral Doppler data.

In accordance with at least one example disclosed herein, a method may include determining whether individual ones of a plurality of cardiac cycles in spectral Doppler data are of high quality or of low quality, based, at least in part on heart rate variability, acquisition quality, or a combination thereof and displaying the spectral Doppler data and a visual indication of one or more of cardiac cycles of the plurality of cardiac cycles determined to be of high quality and cardiac cycles of the plurality of cardiac cycles determined to be of low quality.

In some examples, the method may further include generating a spectrogram based on spectral Doppler data, extracting a spectral envelope of the spectrogram, and determining the plurality of cardiac cycles within at least a portion of the spectral Doppler data based, at least in part, on the spectral envelope. In some examples, determining the plurality of cardiac cycles may include applying an auto-correlation function to the spectral envelope, determining an average cardiac cycle duration based, at least in part, on a first non-zero lag peak of the auto-correlation function, and locating, based at least in part, on the average cardiac cycle duration, a plurality of local maxima and a plurality of local minima in the portion of the spectral Doppler data, wherein individual ones of the plurality of cardiac cycles are located between individual ones of the plurality of local maxima and local minima. In some examples, the locating is further based on a buffer to the average cardiac cycle.

In some examples, a cardiac cycle of the plurality of cardiac cycles is determined to be of low quality based on a comparison of the heart rate variability of the cardiac cycle to a threshold value. In some examples, the heart rate variability of the cardiac cycle is based on a difference between a duration of the cardiac cycle and an average cardiac cycle duration for the plurality of cardiac cycles.

In some examples, a cardiac cycle of the plurality of cardiac cycles is determined to be of low quality based on a comparison of a plurality of cross-correlation coefficients for the cardiac cycle to a threshold value. In some examples, the method may further include calculating the plurality of cross-correlation coefficients by cross-correlating the cardiac cycle to other ones of the plurality of cardiac cycles.

In some examples, the method may further include determining a parameter measurement based on cardiac cycles of the plurality of cardiac cycles determined to be of high quality. In some examples, the method may further include comprising displaying the parameter measurement. In some examples, the parameter may include a resistive index, a peak systolic velocity, an end diastolic velocity, or a combination thereof.

In some examples, the spectral Doppler data comprises pulse wave spectral Doppler data. In accordance with at least one example disclosed herein, a computer readable medium may be encoded with instructions that when executed by at least one processor of a system, may cause the system to perform one or more of the methods disclosed herein.

These and other aspects of the invention will be apparent from and elucidated with reference to the embodiments described hereinafter.

BRIEF DESCRIPTION OF THE DRAWINGS

FIG. 1 is a block diagram of an ultrasound imaging system arranged in accordance with principles of the present disclosure.

FIG. 2A is an example of a Doppler spectrogram in accordance with examples of the present disclosure.

FIG. 2B illustrates a spectral envelope extracted from the spectrogram shown in FIG. 2A in accordance with examples of the present disclosure.

FIG. 3A illustrates an auto-correlation function for the spectral envelope shown in FIG. 2B in accordance with examples of the present disclosure.

FIG. 3B illustrates the detected PSV and EDV for each cardiac cycle of the spectral envelope shown in FIG. 2B in accordance with examples of the present disclosure.

FIG. 4 illustrates plots of values that may be calculated based on an analysis of the spectral envelope in accordance with examples of the present disclosure.

FIG. 5A illustrates a plot of the instantaneous cardiac cycle difference based on the cardiac cycle durations shown in FIG. 4 in accordance with examples of the present disclosure.

FIG. 5B illustrates a plot of the RI values for all of the cardiac cycles based on the values of the PSV and EDV shown in FIG. 4 in accordance with examples of the present disclosure.

FIG. 6 is an example of an output of a display in accordance with examples of the present disclosure.

FIG. 7 is another example of an output of a display in accordance with examples of the present disclosure.

FIG. 8 is a further example of an output of a display in accordance with examples of the present disclosure.

FIG. 9 is a flow chart of a method in accordance with examples of the present disclosure.

FIG. 10 is a block diagram illustrating an example processor in accordance with principles of the present disclosure.

DETAILED DESCRIPTION OF EMBODIMENTS

The following description of certain examples is merely exemplary in nature and is in no way intended to limit the invention or its applications or uses. In the following detailed description of embodiments, reference is made to the accompanying drawings in which are shown by way of illustration specific examples in which the described apparatuses, systems and methods may be practiced. These examples are described in sufficient detail to enable those skilled in the art to practice the presently disclosed apparatuses, systems and methods, and it is to be understood that other examples may be utilized, and that structural and logical changes may be made without departing from the scope of the present disclosure. Moreover, for the purpose of clarity, detailed descriptions of certain features will not be discussed when they would be apparent to those with skill in the art so as not to obscure the description of the present system. The following detailed description is therefore not to be taken in a limiting sense, and the scope of the present system is defined only by the appended claims.

Spectral Doppler, such as spectral pulse wave (PW) Doppler, refers to spectral analysis of Doppler data. A user may select a region of interest (ROI) within a B-mode image for performing Doppler imaging. In some applications, the user may select the ROI for color flow Doppler imaging and then select a sub-region within the ROI for acquiring spectral Doppler data. Several transmit-receive events are performed in the ROI or sub-region to acquire spectral Doppler data. The acquired spectral Doppler data may be analyzed, for example, by performing a Fast Fourier Transform (FFT), to provide a spectrum of frequencies present in the spectral Doppler data. The frequencies may be converted to velocities, which may correspond to velocities of fluid flow in the ROI or sub-region, for example, velocities of blood in a blood vessel. The velocity in the ROI may be plotted over time as a spectrogram. The spectrogram may be analyzed qualitatively or quantitatively, such as for calculating a RI as provided in Equation 1, for diagnosing and/or grading a medical condition. While operating in the PW Doppler mode, the user must maintain a position of the transducer array. Movement of a subject or the probe may affect the PW signal, which in turn may affect the spectrogram and results derived from the spectrogram, such as values for the RI. Unreliable RI values may lead to misdiagnosis and/or grading. For example, in carotid Doppler imaging, inaccurate RI values may lead to over- or under-estimating the subject's risk factors for stroke.

Accuracy of key Doppler parameter measurement may be dependent on acquired Doppler spectrum quality. According to embodiments of the present disclosure, an automatic approach for measuring/calculating parameters (e.g., RI) from a Doppler spectrum (e.g., PW Doppler spectrum) and quality evaluation method is disclosed which may automatically identify portions of the spectrogram associated with (a) low quality Doppler signal and/or (b) cardiac cycles with high heart rate variability (HRV). The techniques may not require an ECG signal. Portions of the spectrogram associated with (a) and/or (b) may be excluded from calculating measurements for one or more parameters, such as the RI. Excluded cardiac cycles may be referred to as "low quality" cardiac cycles. The "low quality" may be due to high HRV and/or motion from either operator or subject (e.g., patient) during the ultrasound examination. In some embodiments, an indication on a display of an ultrasound imaging system may show the user which portions of the spectrogram have been excluded from the analysis. The features of the present disclosure may provide more consistent and/or reliable measurements of parameters derived from the spectrogram. The features of the present disclosure may provide feedback to users as to the quality of the cardiac cycles.

As used herein, a cardiac cycle refers to a sequence of electrical and mechanical events that are performed by the heart during a heartbeat. The period of time over which the sequence of events occurs is referred to a duration of the cardiac cycle. As is generally known, the cardiac cycle includes two phases: diastole and systole. The Peak Systolic Velocity (PSV) is the maximum blood flow velocity measured at a location (e.g., the carotid artery) during the systolic phase of the cardiac cycle. The End Diastolic Velocity (EDV) is the minimum blood flow velocity measured at the location during the diastolic phase of the cardiac cycle.

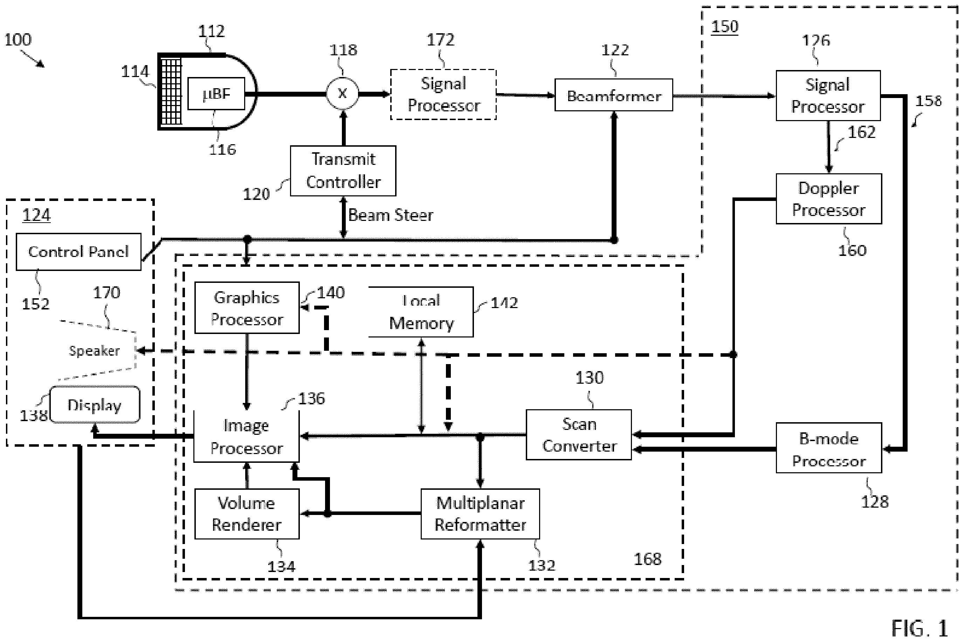

FIG. 1 shows a block diagram of an ultrasound imaging system 100 constructed in accordance with the principles of the present disclosure. An ultrasound imaging system 100 according to the present disclosure may include a transducer array 114, which may be included in an ultrasound probe 112, for example an external probe or an internal probe. The transducer array 114 is configured to transmit ultrasound signals (e.g., beams, waves) and receive echoes (e.g., received ultrasound signals) responsive to the transmitted ultrasound signals. A variety of transducer arrays may be used, e.g., linear arrays, curved arrays, or phased arrays. The transducer array 114, for example, can include a two-dimensional array (as shown) of transducer elements capable of scanning in both elevation and azimuth dimensions for 2D and/or 3D imaging. As is generally known, the axial direction is the direction normal to the face of the array (in the case of a phased or linear array the axial direction may be steered away from normal to the array face and, in the case of a curved array the axial directions fan out), the azimuthal direction is defined generally by the longitudinal dimension of the array (normal to the axial), and the elevation direction is transverse to the azimuthal direction.

In some examples, the transducer array 114 may be coupled to a microbeamformer 116, which may be located in the ultrasound probe 112, and which may control the transmission and reception of signals by the transducer elements in the array 114. In some examples, the microbeamformer 116 may control the transmission and reception of signals by active elements in the array 114 (e.g., an active subset of elements of the array that define the active aperture at any given time).

In some examples, the microbeamformer 116 may be coupled, e.g., by a probe cable or wirelessly, to a transmit/receive (T/R) switch 118, which switches between transmission and reception and protects the main beamformer 122 from high energy transmit signals. In some examples, for example in portable ultrasound systems, the T/R switch 118 and other elements in the system can be included in the ultrasound probe 112 rather than in the ultrasound system base/host, which may house the image processing electronics. An ultrasound system base/host typically includes software and hardware components including circuitry for signal processing and image data generation as well as executable instructions for providing a user interface.

The transmission of ultrasonic signals from the transducer array 114 under control of the microbeamformer 116 is directed by the transmit controller 120, which may be coupled to the T/R switch 118 and a main beamformer 122. The transmit controller 120 may control the direction in which beams are steered. Beams may be steered straight ahead from (orthogonal to) the transducer array 114, or at different angles for a wider field of view. The transmit controller 120 may also be coupled to a user interface 124 and receive input from the user's operation of a user control. The user interface 124 may include one or more input devices such as a control panel 152, which may include one or more mechanical controls (e.g., buttons, encoders, etc.), touch sensitive controls (e.g., a trackpad, a touchscreen, or the like), and/or other known input devices.

In some examples, the partially beamformed signals produced by the microbeamformer 116 may be coupled to a main beamformer 122 where partially beamformed signals from individual patches of transducer elements may be combined into a fully beamformed signal. In some examples, microbeamformer 116 is omitted, and the transducer array 114 is under the control of the main beamformer 122 and main beamformer 122 performs all beamforming of signals. In examples with and without the microbeamformer 116, the beamformed signals of main beamformer 122 are coupled to processing circuitry 150, which may include one or more processors (e.g., a signal processor 126, a B-mode processor 128, a Doppler processor 160, and one or more image generation and processing components 168) configured to produce an ultrasound image from the beamformed signals (i.e., beamformed radiofrequency (RF) data).

The signal processor 126 may process the received beamformed RF data in various ways, such as bandpass filtering, decimation, I (in-phase) and Q (quadrature-phase) complex component separation, and harmonic signal separation. The signal processor 126 may also perform additional signal enhancement such as speckle reduction, signal compounding, and electronic noise elimination. The processed signals (also referred to as I and Q components or IQ signals) may be coupled to additional downstream signal processing circuits for image generation. The IQ signals may be coupled to a plurality of signal paths within the system, each of which may be associated with a specific arrangement of signal processing components suitable for generating different types of image data (e.g., B-mode image data, contrast image data, Doppler image data). For example, the system 100 may include a B-mode signal path 158 which couples the signals from the signal processor 126 to a B-mode processor 128 for producing B-mode image data. The B-mode processor 128 can employ amplitude detection for the imaging of organ structures within the body.

Although the signal processor 126 is described as after the main beamformer 122, in other examples, the signal processor 126 may receive and process signals from the transducer array 114 prior to beamforming and provide the processed signals to the main beamformer 122. In some examples, the system 100 may include two signal processors 126 and 172, where signal processor 172 receives and process signals received from the transducer array 114 and signal processor 126 receives and process signals received from the main beamformer 122.

In some examples, the system may include a Doppler signal path 162 which couples the output from the signal processor 126 to a Doppler processor 160. The Doppler processor 160 may be configured to estimate the Doppler shift and generate Doppler image data. The Doppler image data may include color data which is then overlaid with B-mode (e.g., grayscale) image data for display. The Doppler processor 160 may be configured to filter out unwanted signals (e.g., noise or clutter associated with stationary or slow-moving tissue), for example using a wall filter. The Doppler processor 160 may be further configured to estimate velocity and power in accordance with known techniques. For example, the Doppler processor may include a Doppler estimator such as an auto-correlator, in which velocity (Doppler frequency, color Doppler) estimation is based on the argument of the lag-one (R1) autocorrelation function and Doppler power estimation is based on the magnitude of the lag-zero (R0) autocorrelation function. Motion can also be estimated by known phase-domain (for example, parametric frequency estimators such as MUSIC (Multiple Signal Classifier), ESPRIT (Estimation of Signal Parameters via Rotational Invariant Techniques), etc.) or time-domain (for example, cross-correlation) signal processing techniques. Other estimators related to the temporal or spatial distributions of velocity such as estimators of acceleration or temporal and/or spatial velocity derivatives can be used instead of or in addition to velocity estimators.

In some examples, such as for spectral Doppler, a fast Fourier transform (FFT) may be performed on the Doppler data to provide a spectrum of frequencies included in the Doppler data within one or more time periods. Various types of spectral Doppler may be used, such as pulse wave (PW) Doppler. The frequencies may be converted into velocities. In some examples, the velocity and power estimates may undergo further threshold detection to further reduce noise, as well as segmentation and post-processing such as filling and smoothing. As disclosed herein, the Doppler processor 160 may calculate various measurements based on the spectral Doppler data including the RI, the PSV, and/or the EDV. Additionally, the Doppler processor 160 may determine a quality of a cardiac cycle. The quality may be based on quality of the Doppler spectral signal, heart rate variability, or a combination thereof.

In some examples, velocity and power estimates may then be mapped to a desired range of display colors and/or intensities in accordance with a color and/or intensity map. The color and/or intensity data, also referred to as Doppler image data, may then be coupled to a scan converter 130, where the Doppler image data may be converted to a desired image format and overlaid on a B-mode image of the tissue structure to form a color Doppler or a power Doppler image. For example, Doppler image data may be overlaid on a B-mode image of tissue structure.

In some examples, the velocity data derived from the Doppler image data (e.g., data derived from PW Doppler) may be plotted over time to provide a spectrogram. The spectrogram may be displayed concurrently with a B-mode image, color Doppler image data, power Doppler image data, or combinations thereof. In other examples, only the spectrogram may be displayed. In some examples, the frequency data used to generate the velocity data may be used to generate audible sounds for output by a speaker 170. Low frequency sounds are associated with slow (low) velocity flows and high frequency sounds are associated with fast (high) velocity flows.

The signals produced by the B-mode processor 128 and/or Doppler processor 160 may be coupled to a scan converter 130 and/or a multiplanar reformatter 132. The scan converter 130 may be configured to arrange the echo signals from the spatial relationship in which they were received to a desired image format. For instance, the scan converter 130 may arrange the echo signal into a two-dimensional (2D) sector-shaped format, or a pyramidal or otherwise shaped three-dimensional (3D) format. The multiplanar reformatter 132 can convert echoes which are received from points in a common plane in a volumetric region of the body into an ultrasonic image (e.g., a B-mode image) of that plane, for example as described in U.S. 6,443,896 (Detmer). The scan converter 130 and multiplanar reformatter 132 may be implemented as one or more processors in some examples.

A volume renderer 134 may generate an image (also referred to as a projection, render, or rendering) of the 3D dataset as viewed from a given reference point, e.g., as described in U.S. 6,530,885 (Entrekin et al.). The volume renderer 134 may be implemented as one or more processors. The volume renderer 134 may generate a render, such as a positive render or a negative render, by any known or future known technique such as surface rendering and maximum intensity rendering. Although shown in FIG. 1 as receiving data from the multiplanar reformatter 132, in some examples, the volume renderer 134 may receive data from the scan converter 130.

Output (e.g., B-mode images, Doppler images) from the scan converter 130, the multiplanar reformatter 132, and/or the volume renderer 134 may be coupled to an image processor 136 for further enhancement, buffering and temporary storage before being displayed on an image display 138. In some examples, some Doppler data, such as spectral Doppler data that may include frequencies and/or velocities plotted over time may be provided directly from the Doppler processor 160 to the image processor 136 for generating a spectrogram or other desired data output formats. In some examples, parameters measured based on the spectral Doppler data such as a quality indicator, e.g., RI, PSV, and/or EDV, may be computed by the Doppler processor 160 and provided to the image processor 136 and/or graphics processor 140 for display. A graphics processor 140 may generate graphic overlays for display with images, such as the accumulation image generated by the image processor 136. These graphic overlays can contain, e.g., standard identifying information such as patient name, date and time of the image, imaging parameters, and the like. For these purposes the graphics processor may be configured to receive input from the user interface 124, such as a typed patient name or other annotations. The user interface 124 can also be coupled to the multiplanar reformatter 132 for selection and control of a display of multiple multiplanar reformatted (MPR) images.

The system 100 may include a memory 142. The memory 142 may be implemented as any suitable non-transitory computer readable medium or media (e.g., flash drive, disk drive, dynamic random-access memory (DRAM)) or may be on the cloud. The memory 142 may store data generated by the system 100 including B-mode images, Doppler images, instructions capable of being executed by one or more of the processors included in the system 100 (e.g., Doppler processor 160, image processor 136), inputs provided by a user via the user interface 124, or any other information necessary for the operation of the system 100.

As mentioned previously, the system 100 includes a user interface 124. The user interface 124 may include a display 138 and a control panel 152. The display 138 may include a display device implemented using a variety of known display technologies, such as LCD, LED, OLED, or plasma display technology. In some examples, the display 138 may comprise multiple displays. The control panel 152 may be configured to receive user inputs (e.g., imaging mode, selection of ROI in image). The control panel 152 may include one or more hard controls (e.g., buttons, knobs, dials, encoders, mouse, trackball or others). The control panel 152 may additionally or alternatively include soft controls (e.g., GUI control elements or simply, GUI controls) provided on a touch sensitive display. Display 138 may be a touch sensitive display that includes one or more soft controls of the control panel 152. The user interface 124 may also include a speaker 170 for providing audio signals to the user. For example, speaker 170 may provide audio signals received from the Doppler processor 160, which may be associated with Doppler frequencies detected by the Doppler processor 160.

In some examples, various components shown in FIG. 1 may be combined. For instance, the image processor 136 and graphics processor 140 may be implemented as a single processor. In another example, the scan converter 130 and multiplanar reformatter 132 may be implemented as a single processor. In some examples, various components shown in FIG. 1 may be implemented as separate components. For example, Doppler processor 160 may be implemented as multiple processors. In some examples, the multiple processors may perform different tasks (e.g., spectral Doppler, power Doppler, etc.). In another example, the memory 142 may include multiple memories which may be the same or different memory types (e.g., flash, DRAM).

In some examples, one or more of the various processors shown in FIG. 1 may be implemented by general purpose processors and/or microprocessors configured to perform the specified tasks. For example, the processors may be configured by instructions stored in a computer readable memory (e.g., memory 142) which are executed by the processors to perform the specified tasks. In some examples, one or more of the various processors may be implemented as application specific circuits (ASICs). In some examples, one or more of the various processors (e.g., image processor 136) may be implemented with one or more graphical processing units (GPUs).

While the system 100 is described as an ultrasound imaging system, in some embodiments, some or all of the of the features of the present disclosure may be performed by a system including only some of the components of the ultrasound imaging system 100. For example, the system 100 may only include processing components 168 and user interface 124, and/or may further include doppler processor 160. In these embodiments, the system 100 may receive Doppler data from another source. Other sources include, but are not limited to, another ultrasound imaging system, another computing device, a computer readable medium, a cloud computing system, a network, and a picture archiving and communication system (PACS). The received Doppler data may be stored in the system 100, for example, in memory 142. In some embodiments, the Doppler data may have been previously acquired. That is, embodiments of the present disclosure may be practiced after an ultrasound exam instead of or in addition to during the ultrasound exam.

According to embodiments of the present disclosure, a Doppler spectrum signal with a defined number of segments or duration (e.g. 3-5 cardiac cycles or equivalent time duration) may be analyzed by one or more processors of the ultrasound imaging system 100, for example, by Doppler processor 160. In some examples, the number of segments or duration may be selected by a user via the user interface 124. In some examples, the number of segments or duration may be selected automatically by ultrasound imaging system 100. The one or more processors may perform an auto-correction function to estimate average cardiac duration T0, which may be the first non-zero lag corresponding to the maximum of autocorrelation function).

The one or more processors may search for the Doppler spectral signal local maximum (which may correspond to PSV) and local minimum (which may correspond to EDV) using the averaged cardiac duration T0. In some examples, additional time may be added to the average cardiac time to provide a buffer. The buffer time may accommodate heart rate variation among cycles.

The one or more processors may compute Doppler spectral signal quality indices using cross-correlation (CC) between any paired cardiac cycles. The one or more processors may evaluate each cardiac cycle using the quality index (e.g., a pre-determined CC coefficient threshold) and a heart rate variability index to identify the cardiac cycles (if any) that fall into one or both categories of a) low-quality cardiac cycles or b) cardiac cycles with high HRV.

The one or more processors may calculate an average parameter measurement (e.g., the RI) by excluding the outlier cardiac cycles (e.g., the ones that fell into one or both of categories a and b).

The ultrasound imaging system may provide on a display, such as the display 138, a dynamic 2D Doppler spectrogram and indicate the detection of cardiac cycles in high quality and low quality. For example, the Doppler signals in the spectrogram corresponding to high quality cardiac cycles may be shown in a different color, line texture (e.g., dashes vs. solid), line thickness, or a combination thereof, compared to Doppler signals corresponding to low quality cardiac cycles. In some examples, different portions of the spectrogram may be shaded, different colors, included in a box, or a combination thereof, depending on whether the cardiac cycles in those portions of the spectrogram are of high quality or low quality. In some examples, the average parameter measurement may be displayed (e.g., text). In some examples, the detected location(s) of the PSV and the EDV in the spectrogram may be displayed (e.g., vertical lines at the time point(s) in the spectrogram).

The features of the present disclosure will be described in further detail using the RI as an example of a parameter measured from the Doppler data. However, the RI is used for illustrative purposes and the features of the disclosure are not limited to this parameter.

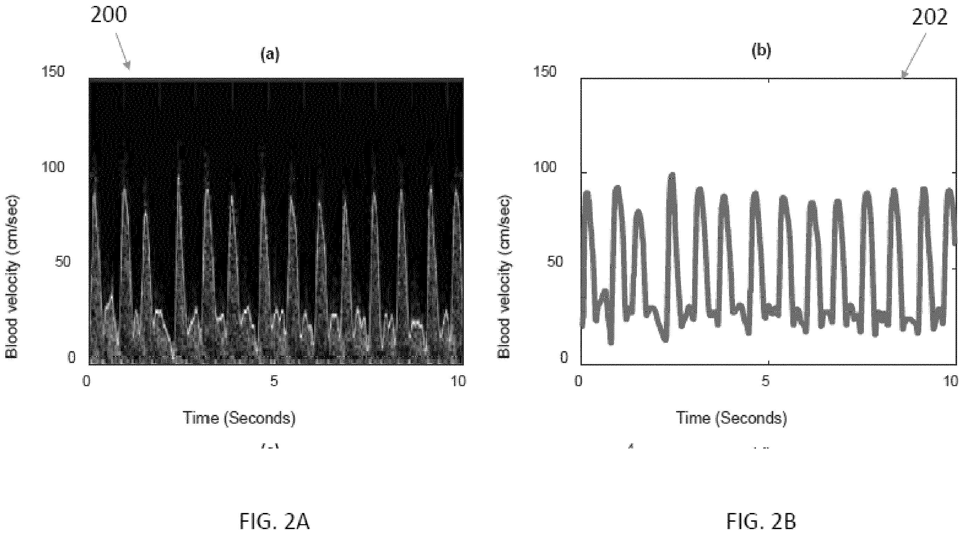

FIG. 2A is an example of a Doppler spectrogram in accordance with examples of the present disclosure. The spectrogram 200 was generated using PW spectral Doppler from a carotid artery. The spectrogram 200 plots blood velocity over time. The Doppler signals may have been acquired using an ultrasound probe, such as ultrasound probe 112. Transducer elements of an array, such as array 114, may have transmitted ultrasound signals into a subject and received corresponding echo signals from the subject. The received signals may have been processed by one or more processors, such as signal processor 172, signal processor 126, and/or Doppler processor 160 to derive a Doppler spectrum from the signals. In some examples, the one or more processors may have removed artifacts (e.g., granular noise and/or speckle noise) from the Doppler signals. The Doppler signals may be processed to generate the spectrogram 200. In some examples, the Doppler signals may be processed as a 2D grey-scale image to generate the spectrogram 200. The one or more processors may extract a spectral envelope signal from the Doppler spectrogram. FIG. 2B illustrates a spectral envelope 202 extracted from the spectrogram shown in FIG. 2A in accordance with examples of the present disclosure.

Once the spectral envelope 202 has been extracted, the one or more processors may select all or a portion of the spectral envelope 202 for further analysis. In some examples, the one or more processors may select a portion that includes three or more cardiac cycles. In some examples, the portion may be determined based, at least in part, on average cardiac cycle durations for a subject population (e.g., all adults, adult males between 40-45 years old), previously acquired ECG data, user input indicating average cardiac cycle duration for the subject, or a combination thereof. In the example shown in FIGS 2A and 2B, the portions of the spectrogram 200 and spectral envelope 202 include approximately 13 cardiac cycles. The subject from which the spectrogram 200 was acquired had relatively large heart rate variability.

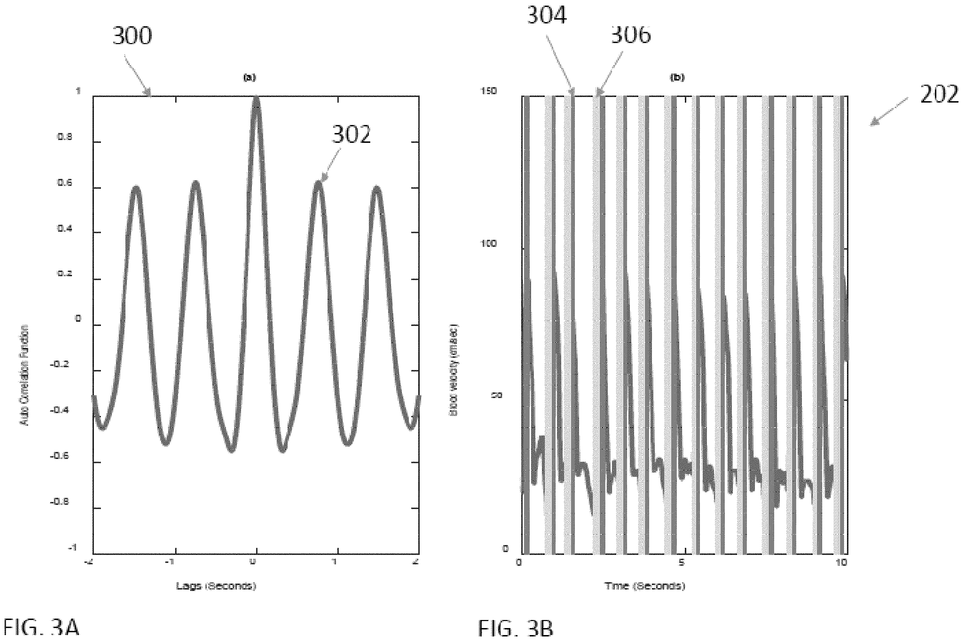

An auto-correlation function may be applied to the selected portion of the spectral envelope 202 to estimate the average cardiac cycle duration. FIG. 3A illustrates an auto-correlation function result 300 for the spectral envelope shown in FIG. 2B in accordance with examples of the present disclosure. The average cardiac cycle duration may be determined based on a location of a first non-zero lag peak 302 (T0) of the auto-correlation function result 300. In the example shown in FIG. 3A, the average cardiac cycle is 0.7553 seconds. In some examples, searching for the first non-zero lag peak 302 may be limited to a range. For example, a range of [0.375 to 1.5] seconds which corresponds to a heart rate range of [40 to 160] beats per minute (BPM).

Once the average cardiac cycle duration is determined, the spectral envelope 202 may be searched for local maximum and local minimum. The local maxima may correspond to the PSV and local minima may correspond to the EDV. In some examples, the search for maxima and minima for each cardiac cycle may be limited to a range. For example, a range of T0 plus a buffer (e.g. T0 $\pm$ 20% T0). FIG. 3B illustrates the detected PSV 304 and EDV 306 for each cardiac cycle of the spectral envelope 202 in accordance with examples of the present disclosure.

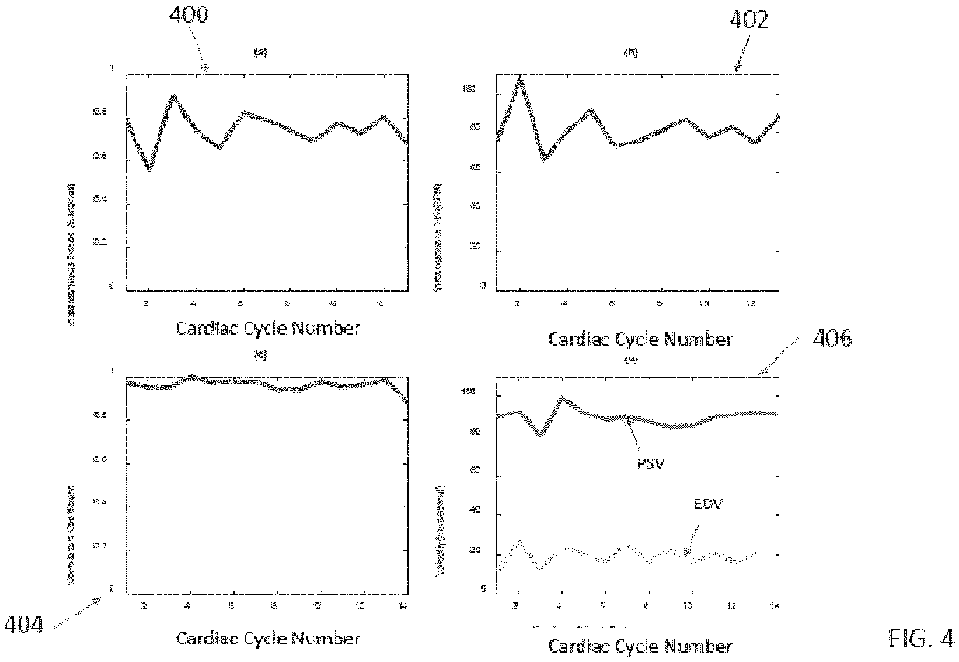

FIG. 4 illustrates plots of values that may be calculated based on an analysis of the spectral envelope in accordance with examples of the present disclosure. In some examples, based, at least in part, on a time period between maxima (PSV) or minima (EDV) of sequential heart cycles, the instantaneous cardiac cycle duration (e.g., instantaneous period) may be estimated for each cardiac cycle. In some examples, the instantaneous cardiac cycle duration may be estimated based, at least in part, on a time period between the maxima and minima of a cardiac cycle. Plot 400 of FIG. 4 illustrates an estimated cardiac cycle duration for each cardiac cycle of the spectral envelope 202. Based on the estimated cardiac cycle duration, the corresponding estimated heart rate may be calculated for each cardiac cycle. Plot 402 of FIG. 4 illustrates the heart rate estimated for each cardiac cycle.

In some examples, the cardiac cycles of the spectral envelope may be segmented from one another based on the determined PSV and EDV. Once segmented, the cardiac cycles may be cross-correlated to one another. Plot 404 displays the cross-correlation coefficient between individual ones of the cardiac cycles and the fourth cardiac cycle shown in spectral envelope 202. Plot 406 illustrates the values of the PSV and EDV detected for each cardiac cycle. The duration of each cardiac cycle and/or the cross-cross correlations may be used to determine the quality of the cardiac cycle. In some examples, cardiac cycles determined to be of high quality may be used for calculating the PSV, the EDV, and/or the RI. Low quality cardiac cycles may be excluded from the calculations.

Determining whether a cardiac cycle is of low quality or of high quality may be based on one or more factors. In some examples, cardiac cycles having HRV may be determined to be of low quality as cardiac cycles occurring during a period of high variability in heart rate may have abnormal PSV and EDV values that do not reflect the typical values for a subject. In some examples, cardiac cycles having poor acquisition quality (e.g., poor Doppler spectral signal) may be determined to be of low quality as the poor acquisition may make determining parameters such as the PSV and/or the EDV impossible or unreliable due to noise, low amplitude, etc. In some examples, a cardiac cycle may be excluded as low quality when it has a HRV, poor acquisition quality, or both.

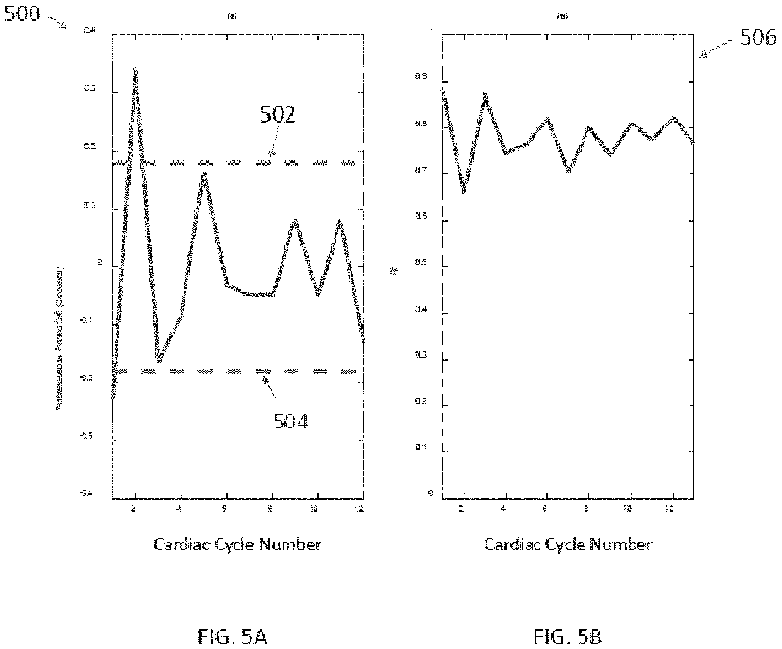

HRV may be caused due to a physiological condition of the subject or may be an artifact due to poor Doppler signal acquisition. Regardless of whether the HRV is due to "true" heart rate irregularities or artifacts, both shall be referred to as HRV. In some examples, HRV may be determined for a cardiac cycle by taking a difference between the estimated instantaneous cardiac cycle duration (shown in plot 400) and an average of the cardiac cycle durations. FIG. 5A illustrates a plot 500 of the instantaneous cardiac cycle difference based on the cardiac cycle durations shown in plot 400 of FIG. 4. Cardiac cycles having a duration that is significantly greater or less than the average duration may indicate a HRV, and the cardiac cycle may be determined to be of low quality. In some examples, cardiac cycles having a difference in duration greater and/or less than threshold values may be determined to be of low quality. In some examples, the threshold values may be based, at least in part, on a standard deviation of the cardiac cycle durations. In the example shown in FIG. 5A, dashed lines 502 and 504 indicate an acceptable range of differences in cardiac cycle duration is between approximately +/-0.19s. Cardiac cycles falling outside this range (e.g., the first and second cardiac cycles) may be determined to be of low quality and excluded from subsequent analysis.

FIG. 5B illustrates a plot of RI values for all of the cardiac cycles based on the values of PSV and EDV shown in plot 406. As shown in plot 506, the values for RI calculated for the first three cardiac cycles that have high HRV have a greater variation than RI values for later cardiac cycles that have less HRV. Accordingly, if the final RI measurement (which is an average of the RI values found for the cardiac cycles) included values of RI for the early cardiac cycles, the final RI measurement may not be accurate. Accordingly, removing the early cardiac cycles with high HRV from the calculation of the final measurement of the RI may be desirable.

Cardiac cycles having poor acquisition quality may be determined based on the cross-correlation. Cross-correlation coefficients (CC) between a cardiac cycle and all of the other cardiac cycles of the spectral envelope may be calculated. An example of calculating the CC for a cardiac cycle is shown in plot 404 of FIG. 4. Table 1 provides example cross-correlation coefficient values for a subject with high quality Doppler acquisition and Table 2 provides example cross-correlation coefficient values for a subject with low quality Doppler acquisition.

**Table 1: Example CC for High Quality Subject**

| Cycle/Corr Coeff | 1 | 2 | 3 | 4 |
|---|---|---|---|---|
| 1 | 1.0 | 0.868 | 0.755 | 0.87 |

(continued)

| Cycle/Corr Coeff | 1 | 2 | 3 | 4 |
|---|---|---|---|---|
| 2 | 0.887 | 1.0 | 0.816 | 0.858 |
| 3 | 0.80 | 0.823 | 1.0 | 0.736 |
| 4 | 0.85 | 0.829 | 0.89 | 1.0 |

**Table 2: Example CC for Low Quality Subject**

| Cycle/Corr Coeff | 1 | 2 | 3 | 4 |
|---|---|---|---|---|
| 1 | 1.0 | 0.794 | 0.82 | 0.503 |
| 2 | 0.814 | 1.0 | 0.85 | 0.45 |
| 3 | 0.81 | 0.851 | 1.0 | 0.43 |
| 4 | 0.502 | 0.46 | 0.42 | 1.0 |

In some examples, the quality of a cardiac cycle may be based on the comparison of the associated CC values to a threshold value. For example, a cardiac cycle may be determined to be high quality if an average of the associated CC values is equal to or above a threshold value and low quality if an average of the associated CC values are below a threshold value (e.g., 0.6). In some examples, the CC value of a cardiac cycle with itself may be excluded from the calculations (as it would always equal 1.0). As shown in the examples above, all CC values for the high-quality case in Table 1 are greater than 0.7. In contrast, the low-quality case in Table 2 has a subset of CC values that are low (e.g., .503 or below), associated with cardiac cycle 4. Thus, the acquisition quality of cardiac cycle 4 may be poor, and cardiac cycle 4 may be determined to be of low quality.

In some examples, a Doppler spectrogram/cardiac cycle quality index may be computed by evaluating an obtained heart rate and CC values for the cardiac cycles. In some examples, if (1) the obtained heart rate is between 40 to 160 BPM, (2) the CC is greater than a pre-determined threshold (e.g., 0.6), and (3) the instantaneous cardiac period difference is less than the normal values for period difference (e.g., 141 ms plus a standard deviation of 39 ms), then the quality index may be 1 (cardiac cycle is of high quality) and that specific cardiac cycle is suitable for subsequent measurement. If a cardiac cycle is missing one or all of the three factors, the quality index may be 0 (cardiac cycle is of low quality). However, in other examples, the quality index may not be binary.

If no single cardiac cycle is determined to be of high quality, the system may automatically search another portion of the Doppler spectrogram for high quality cardiac cycle data that is stored in a computer memory (e.g., a non-transitory computer readable medium) buffer of the ultrasound imaging system and/or the user may be prompted to select another portion. In some examples, the memory buffer may be included in a local memory, such as memory 142. If the memory buffer is empty, the ultrasound imaging system may prompt the user (e.g., text on display 138, sound from speaker 170) to make a new spectral Doppler acquisition. The process may then be repeated.

If one or more cardiac cycles are determined to be high quality, the RI values for the high-quality cardiac cycles are averaged and provided as the final RI measurement. The final RI measurement may be provided as text on the display. In some examples, the cardiac cycles used to calculate the RI may be indicated on the display, such as by highlighting the portions of the spectrogram (e.g., spectrogram 200) associated with the high-quality cardiac cycles.

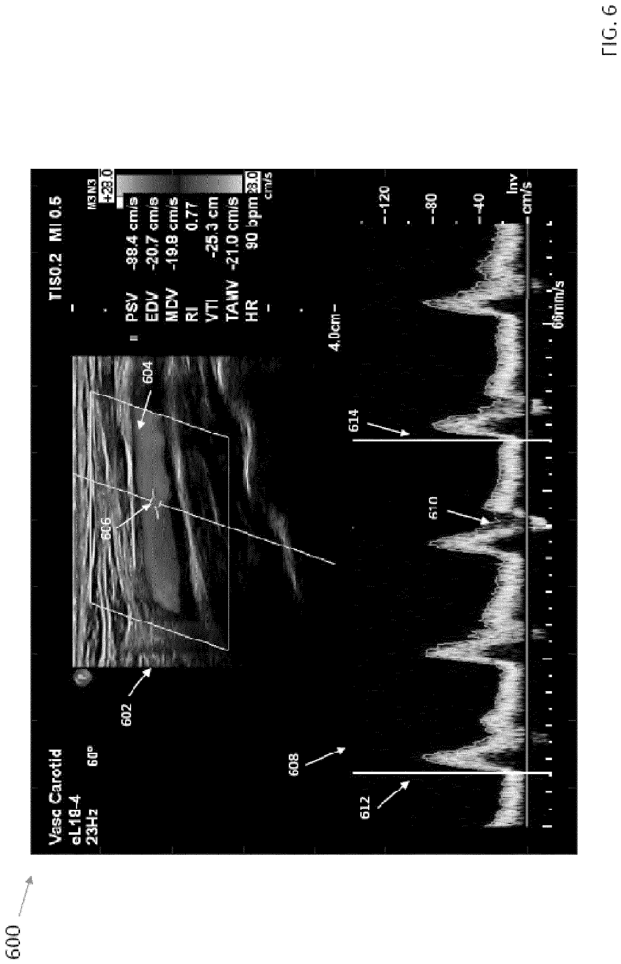

FIG. 6 is an example of an output of a display in accordance with examples of the present disclosure. Output 600 may be provided on a display of an ultrasound imaging system, such as display 138 of ultrasound imaging system 100. Output 600 includes a B-mode image 602 of a portion of a carotid artery with a Doppler color flow image 604 overlay. A cross-hair 606 indicates a region of interest within the Doppler color flow image 604 where spectral PW Doppler signals are acquired. A spectrogram 608 plotting the calculated velocities over time is provided below. A spectral envelope 610 of the spectrogram 608 is also displayed. However, in some examples, the spectral envelope 610 may not be displayed. In the upper right hand region of output 600, text indicating the values calculated for various parameters such as the PSV, EDV, and RI are shown. In some examples, the values may be based on averages of the cardiac cycles within the portion of the spectrogram 608 located between vertical lines 612 and 614. In some examples, the portion selected via the vertical lines 612, 614 may have been selected by a user. In other examples, the portion may have been automatically selected by the ultrasound imaging system. In some examples, one or more of the parameters shown in output 600 may reflect an average value across the cardiac cycles between vertical lines 612, 614. In the example shown in FIG. 6, all of the cardiac cycles in the spectrogram 608 are of high quality. Accordingly, all of the cardiac cycles may be used to measure the parameters.

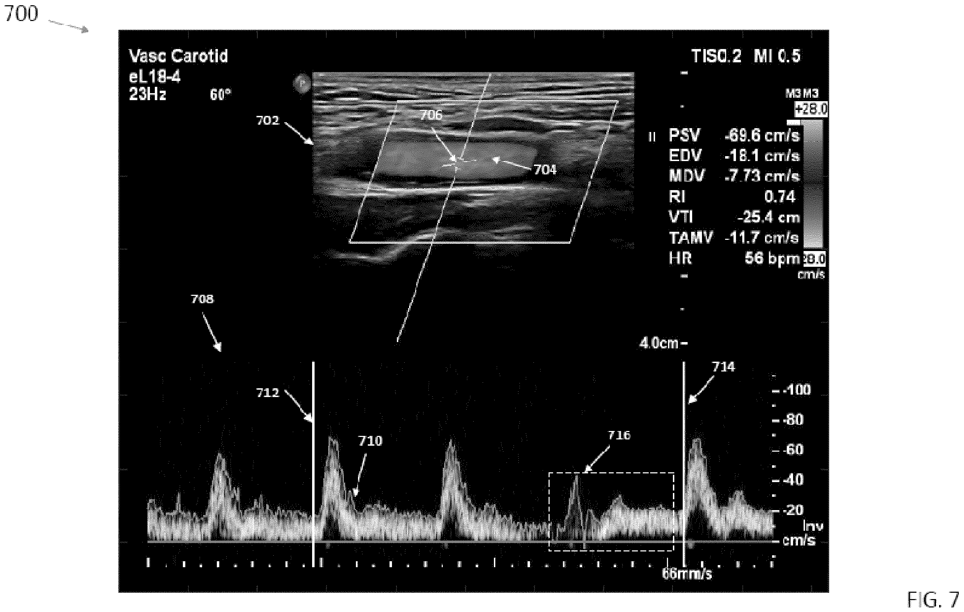

FIG. 7 is another example of an output of a display in accordance with examples of the present disclosure. Output 700 may be provided on a display of an ultrasound imaging system, such as display 138 of ultrasound imaging system 100. Similar to the example shown in FIG. 6, output 700 includes a B-mode image 702 of a portion of a carotid artery with a Doppler color flow image 704 overlay. A cross-hair 706 indicates a region of interest within the Doppler color flow image 704 where spectral PW Doppler signals are acquired. A spectrogram 708 plotting the calculated velocities over time is provided below. A spectral envelope 710 of the spectrogram 708 is also displayed. However, in some examples, the spectral envelope 710 may not be displayed. In the upper right hand region of output 700, text indicating the values calculated for various parameters such as the PSV, EDV, and RI are shown. In some examples, the values may be based on averages of the cardiac cycles within the portion of the spectrogram 708 located between vertical lines 712 and 714. In some examples, the portion selected via the vertical lines 712, 714 may have been selected by a user. In other examples, the portion may have been automatically selected by the ultrasound imaging system. In some examples, one or more of the parameters shown in output 700 may reflect an average value across the cardiac cycles between vertical lines 712, 714.

Both spectrogram 608 and spectrogram 708 were acquired from the same subject during the same exam of the carotid artery. However, in spectrogram 708, a low-quality cardiac cycle indicated by box 716 is included in the calculation of the parameters. Note how the inclusion of a single poor quality cardiac cycle causes the RI to change from 0.77 to 0.74. In some applications, this difference in RI may result in an error in diagnosis and/or grading of the subject's condition. Accordingly, the cardiac cycle indicated by box 716 should be excluded from the calculations to ensure a correct result is acquired, such as the one shown in FIG. 6. The low quality cardiac cycle may be detected automatically and excluded as described with reference to FIGS. 1-5.

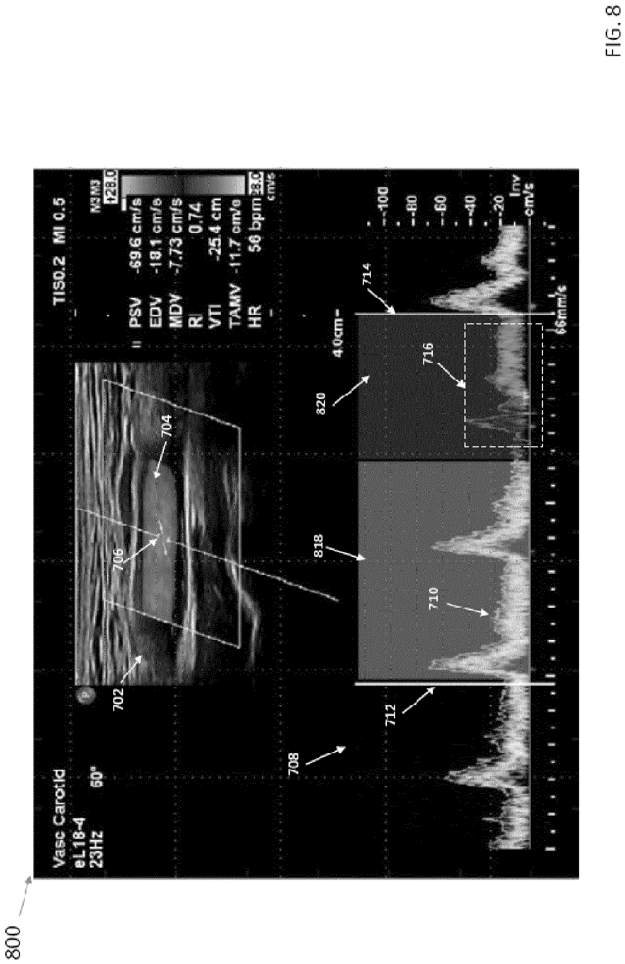

FIG. 8 is a further example of an output of a display in accordance with examples of the present disclosure. Output 800 may be provided on a display of an ultrasound imaging system in some examples, such as display 138 of ultrasound imaging system 100. Output 800 includes the same B-mode image 702, Doppler color flow image 704 overlay, cross-hair 706, spectrogram 708, and spectral envelope 710 as shown in FIG. 7. Additionally, output 800 illustrates an example of providing a visual indication to a user of which cardiac cycles have been determined to be of high quality and which cardiac cycles have been determined to be of low quality. In the example shown in FIG. 8, the high-quality cardiac cycles are included in a highlighted region 818 that has one color (e.g., green), and the low-quality cardiac cycle is in a highlighted region 820 that has another color (e.g., red). Thus, the user can see which cardiac cycles are used and not used for calculating the various measured parameters (e.g., RI). In some examples, if the user disagrees with the determinations made by the ultrasound imaging system, the user may drag the vertical lines 712, 714 to select additional and/or different portions of the spectrogram 708 for analysis and/or adjust the size and/or position of highlighted region 818 and/or 820 to change which cardiac cycles are included or excluded.

While highlighting the cardiac cycles in different colors is shown in FIG. 8, in other examples, other visual indications may be used. For example, the weight of the lines of the spectrogram 708 for the high-quality and low-quality cardiac cycles may be different, the texture of the lines may be different (e.g., solid, dashed, dotted), the colors of the lines may be different, the brightness of the lines may be different, or a combination thereof. In another example, high- and low-quality cardiac cycles may be surrounded by boxes with different line types, textures, and/or colors.

As described with reference to Figs. 1-8, an ultrasound imaging system, such as ultrasound imaging system 100 may include an ultrasound probe, such as ultrasound probe 112 to acquire spectral Doppler data, such as PW Doppler data. The ultrasound imaging system may include a display, such as display 138, and one or more processors, such as signal processors 172, 126, Doppler processor 160, image processor 136, and/or graphics processor 140. The at least one processor may determine whether cardiac cycles in the spectral Doppler data are high quality or low quality, based, at least in part on the HRV, acquisition quality, or a combination thereof, and generate display data based on the determination. The display may provide a visual indication of cardiac cycles of the plurality of cardiac cycles determined to be of high quality and cardiac cycles of the plurality of cardiac cycles determined to be of low quality based on the display data.

The display may provide the spectral Doppler data as a spectrogram, for example as shown in FIGS. 6-8. In some examples, the visual indication includes highlighting the cardiac cycles of the plurality of cardiac cycles determined to be of high quality in a first color and highlighting the cardiac cycles of the plurality of cardiac cycles determined to be of low quality in a second color different than the first color. For example, as shown in FIG. 8. However, in some examples, the visual indication may include displaying a portion of the spectrogram associated with the cardiac cycles of the plurality of cardiac cycles determined to be of high quality in a different color, a different line weight, a different line texture, or a combination thereof than the cardiac cycles of the plurality of cardiac cycles determined to be of low quality.

In some examples, the at least one processor may determine a parameter measurement based on cardiac cycles of the plurality of cardiac cycles determined to be of high quality. In some examples, the display may provide the parameter measurement. For example, as shown in the upper right of FIGS. 6-8.

In some examples, the ultrasound imaging system may include a computer memory buffer to store additional Doppler spectral data. In some examples, the buffer may be located in a local memory, such as memory 142. In other examples, the memory buffer may be located remotely or on a cloud. In some examples, when all of the plurality of cardiac cycles are determined to be of low quality, at least one processor may determine whether cardiac cycles in the additional spectral Doppler data are of high quality or low quality. When the buffer is empty, in some examples, the at least one processor may cause the display to prompt a user to acquire the additional Doppler spectral data. However, in other examples, the at least one processor may cause another potion of the user interface to prompt the user to acquire additional data, such as speaker 170.

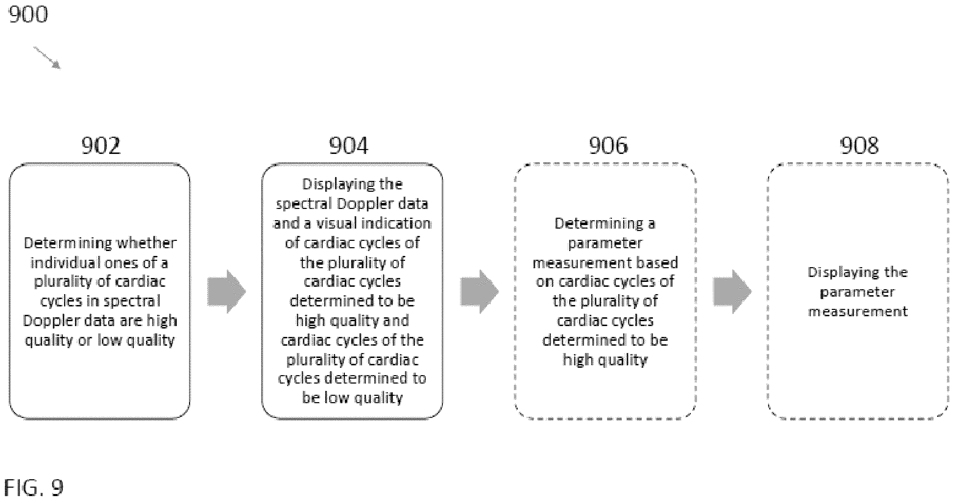

FIG. 9 is a flow chart of a method in accordance with examples of the present disclosure. The method 900 shown in FIG. 9 may be performed in whole or in part by an ultrasound imaging system, such as ultrasound imaging system 100.

At block 902 "determining whether individual ones of a plurality of cardiac cycles in spectral Doppler data are high quality or low quality" may be performed. In some examples, the determination may be based, at least in part on heart rate variability (HRV), acquisition quality, or a combination thereof. In some examples, the determining may be performed by one or more processors such as signal processors 172, 126, Doppler processor 160, image processor 136, and/or graphics processor 140. In some examples, the spectral Doppler data may include pulse wave spectral Doppler data.

In some examples, a cardiac cycle may be determined to be low quality based on a comparison of the heart rate variability of the cardiac cycle to a threshold value. In some examples, the heart rate variability (HRV) of the cardiac cycle is based on a difference between a duration of the cardiac cycle and an average cardiac cycle duration for the plurality of cardiac cycles. For example, as described with reference to FIGS. 4, 5A, and 5B.

In some examples, a cardiac cycle may be determined to be of low quality based on a comparison of cross-correlation coefficients (CCs) for the cardiac cycle to a threshold value. In some examples, method 900 may include calculating the cross-correlation coefficients (CCs) by cross-correlating the cardiac cycle to other cardiac cycles. These examples are described with reference to FIG. 4 and Tables 1 and 2.

At block 904 "displaying the spectral Doppler data and a visual indication of cardiac cycles of the plurality of cardiac cycles determined to be high quality and cardiac cycles of the plurality of cardiac cycles determined to be low quality" may be performed. In some examples, the displaying may be performed by a display, such as display 138. Visual indications may include colors, highlighting, boxes, textures, weights, or a combination thereof, including, but not limited to the example shown in FIG. 8.

In some examples, method 900 may include block 906 where "determining a parameter measurement based on cardiac cycles of the plurality of cardiac cycles determined to be high quality" may be performed. In some examples, method 900 may include block 908 where "displaying the parameter measurement" may be performed. In some examples, the parameter may include a resistive index (RI), a peak systolic velocity (PSV), an end diastolic velocity (EDV), or a combination thereof.

In some examples, method 900 may further include generating a spectrogram based on spectral Doppler data and extracting a spectral envelope of the spectrogram. For example, as described with reference to FIGS. 2A and 2B. In some examples, method 900 may further include determining the cardiac cycles within at least a portion of the spectral Doppler data based, at least in part, on the spectral envelope. In some examples, determining the cardiac cycles may include applying an auto-correlation function to the spectral envelope and determining an average cardiac cycle duration based, at least in part, on a first non-zero lag peak of the auto-correlation function. For example, as described with reference to FIG. 3A. In some examples, determining the cardiac cycles may include locating, based at least in part, on the average cardiac cycle duration, local maxima and local minima in the portion of the spectral envelope. In some examples, individual ones of the plurality of cardiac cycles are located between individual ones of the plurality of local maxima and local minima. For example, as described with reference to FIG. 3B. In some examples, locating the maxima and minima may be further based on a buffer to the average cardiac cycle for example T0 +/- 20%.

In some examples, a computer readable medium (e.g., memory 142) may be encoded with instructions that when executed by at least one processor (e.g., Doppler processor 160) of a system (e.g., system 100), may cause the system to perform some or all of the method 900.

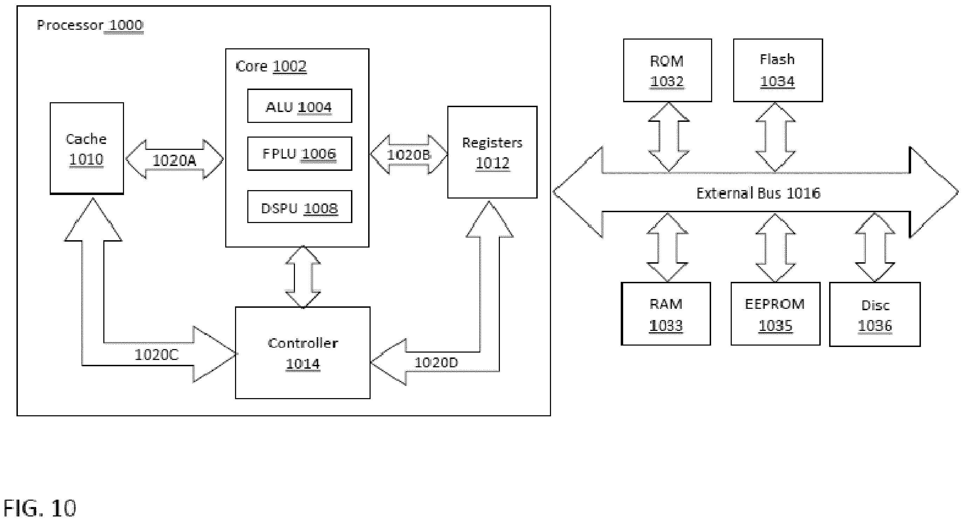

FIG. 10 is a block diagram illustrating an example processor 1000 according to principles of the present disclosure. Processor 1000 may be used to implement one or more processors described herein, for example, image processor 136 and/or Doppler processor 160 shown in FIG. 1. Processor 1000 may be capable of executing computer-readable instructions stored on a non-transitory computer-readable medium in communication with the processor 1000, for example, local memory 142 shown in FIG. 1. Processor 1000 may be any suitable processor type including, but not limited to, a microprocessor, a microcontroller, a digital signal processor (DSP), a field programmable array (FPGA) where the FPGA has been programmed to form a processor, a graphical processing unit (GPU), an application specific circuit (ASIC) where the ASIC has been designed to form a processor, or a combination thereof.

The processor 1000 may include one or more cores 1002. The core 1002 may include one or more arithmetic logic units (ALU) 1004. In some examples, the core 1002 may include a floating-point logic unit (FPLU) 1006 and/or a digital signal processing unit (DSPU) 1008 in addition to or instead of the ALU 1004.

The processor 1000 may include one or more registers 1012 communicatively coupled to the core 1002. The registers 1012 may be implemented using dedicated logic gate circuits (e.g., flip-flops) and/or any memory technology. In some examples the registers 1012 may be implemented using static memory. The register may provide data, instructions and addresses to the core 1002.

In some examples, processor 1000 may include one or more levels of cache memory 1010 communicatively coupled to the core 1002. The cache memory 1010 may provide computer-readable instructions to the core 1002 for execution. The cache memory 1010 may provide data for processing by the core 1002. In some examples, the computer-readable instructions may have been provided to the cache memory 1010 by a local memory, for example, local memory attached to the external bus 1016. The cache memory 1010 may be implemented with any suitable cache memory type, for example, metal-oxide semiconductor (MOS) memory such as static random-access memory (SRAM), dynamic random-access memory (DRAM), and/or any other suitable memory technology.

The processor 1000 may include a controller 1014, which may control input to the processor 1000 from other processors and/or components included in a system (e.g., control panel 152 and scan converter 130 shown in FIG. 1) and/or outputs from the processor 1000 to other processors and/or components included in the system (e.g., display 138 and volume renderer 134 shown in FIG. 1). Controller 1014 may control the data paths in the ALU 1004, FPLU 1006 and/or DSPU 1008. Controller 1014 may be implemented as one or more state machines, data paths and/or dedicated control logic. The gates of controller 1014 may be implemented as standalone gates, FPGA, ASIC or any other suitable technology.

The registers 1012 and the cache memory 1010 may communicate with controller 1014 and core 1002 via internal connections 1020A, 1020B, 1020C and 1020D. Internal connections may be implemented as a bus, multiplexor, crossbar switch, and/or any other suitable connection technology.

Inputs and outputs for the processor 1000 may be provided via a bus 1016, which may include one or more conductive lines. The bus 1016 may be communicatively coupled to one or more components of processor 1000, for example the controller 1014, cache memory 1010, and/or register 1012. The bus 1016 may be coupled to one or more components of the system, such as display 138 and control panel 152 mentioned previously.

The bus 1016 may be coupled to one or more external memories. The external memories may include Read Only Memory (ROM) 1032. ROM 1032 may be a masked ROM, Electronically Programmable Read Only Memory (EPROM) or any other suitable technology. The external memory may include Random Access Memory (RAM) 1033. RAM 1033 may be a static RAM, battery backed up static RAM, Dynamic RAM (DRAM) or any other suitable technology. The external memory may include Electrically Erasable Programmable Read Only Memory (EEPROM) 1035. The external memory may include Flash memory 1034. The external memory may include a magnetic storage device such as disc 1036. In some examples, the external memories may be included in a system, such as ultrasound imaging system 100 shown in FIG. 1, for example local memory 142.

In various examples where components, systems and/or methods are implemented using a programmable device, such as a computer-based system or programmable logic, it should be appreciated that the above-described systems and methods can be implemented using any of various known or later developed programming languages, such as "C", "C++", "FORTRAN", "Pascal", "VHDL" and the like. Accordingly, various storage media, such as magnetic computer disks, optical disks, electronic memories and the like, can be prepared that can contain information that can direct a device, such as a computer, to implement the above-described systems and/or methods. Once an appropriate device has access to the information and programs contained on the storage media, the storage media can provide the information and programs to the device, thus enabling the device to perform functions of the systems and/or methods described herein. For example, if a computer disk containing appropriate materials, such as a source file, an object file, an executable file or the like, were provided to a computer, the computer could receive the information, appropriately configure itself and perform the functions of the various systems and methods outlined in the diagrams and flowcharts above to implement the various functions. That is, the computer could receive various portions of information from the disk relating to different elements of the above-described systems and/or methods, implement the individual systems and/or methods and coordinate the functions of the individual systems and/or methods described above.

In view of this disclosure, it is noted that the various methods and devices described herein can be implemented in hardware, software, and/or firmware. Further, the various methods and parameters are included by way of example only and not in any limiting sense. In view of this disclosure, those of ordinary skill in the art can implement the present teachings in determining their own techniques and needed equipment to affect these techniques, while remaining within the scope of the invention. The functionality of one or more of the processors described herein may be incorporated into a fewer number or a single processing unit (e.g., a CPU) and may be implemented using application specific integrated circuits (ASICs) or general-purpose processing circuits which are programmed responsive to executable instructions to perform the functions described herein.

Although the present system may have been described with particular reference to an ultrasound imaging system, it is also envisioned that the present system can be extended to other medical imaging systems where one or more images are obtained in a systematic manner. Accordingly, the present system may be used to obtain and/or record image information related to, but not limited to renal, testicular, breast, ovarian, uterine, thyroid, hepatic, lung, musculoskeletal, splenic, cardiac, arterial and vascular systems, as well as other imaging applications related to ultrasound-guided interventions. Further, the present system may also include one or more programs which may be used with conventional imaging systems so that they may provide features and advantages of the present system. Certain additional advantages and features of this disclosure may be apparent to those skilled in the art upon studying the disclosure, or may be experienced by persons employing the novel system and method of the present disclosure. Another advantage of the present systems and method may be that conventional medical image systems can be easily upgraded to incorporate the features and advantages of the present systems, devices, and methods.

Of course, it is to be appreciated that any one of the examples, examples or processes described herein may be combined with one or more other examples, examples and/or processes or be separated and/or performed amongst separate devices or device portions in accordance with the present systems, devices and methods.

Finally, the above discussion is intended to be merely illustrative of the present systems and methods and should not be construed as limiting the appended claims to any particular example or group of examples. Thus, while the present system has been described in particular detail with reference to exemplary examples, it should also be appreciated that numerous modifications and alternative examples may be devised by those having ordinary skill in the art without departing from the broader and intended scope of the present systems and methods as set forth in the claims that follow. Accordingly, the specification and drawings are to be regarded in an illustrative manner and are not intended to limit the scope of the appended claims.

In the claims, any reference signs placed between parentheses shall not be construed as limiting the claim. The word "comprising" does not exclude the presence of elements or steps other than those listed in a claim. The word "a" or "an" preceding an element does not exclude the presence of a plurality of such elements.

**Claims**

1. A system (100) comprising:

   a computer readable memory (142) storing spectral Doppler data;
   a display (138); and
   at least one processor (172, 126, 160, 136, 140) configured to:

   determine whether individual ones of a plurality of cardiac cycles in the spectral Doppler data are of high quality or of low quality, based, at least in part on heart rate variability, acquisition quality, or a combination thereof; and
   generate display data based on the determination,

   wherein based on the display data, the display (138) is configured to provide one or more of

> a visual indication of cardiac cycles of the plurality of cardiac cycles determined to be of high quality and
> a visual indication of cardiac cycles of the plurality of cardiac cycles determined to be of low quality.

2. The system (100) of claim 1, wherein the display (138) is configured to provide the spectral Doppler data as a spectrogram (200, 608, 708).

3. The system (100) of claim 2,

> wherein the visual indication comprises highlighting the cardiac cycles of the plurality of cardiac cycles determined to be of high quality in a first color and highlighting the cardiac cycles of the plurality of cardiac cycles determined to be of low quality in a second color different than the first color;
> OR
> wherein the visual indication comprises displaying a portion of the spectrogram associated with the cardiac cycles of the plurality of cardiac cycles determined to be of high quality in a different color, a different line weight, a different line texture, or a combination thereof than the cardiac cycles of the plurality of cardiac cycles determined to be of low quality.

4. The system (100) of any one of claims 1 to 3, wherein the at least one processor (172, 126, 160, 136, 140) is further configured to determine a parameter measurement based on cardiac cycles of the plurality of cardiac cycles determined to be of high quality, and wherein optionally the display (138) is further configured to provide the parameter measurement.

5. The system (100) of any one of claims 1 to 4, further comprising a buffer (142) configured to store additional Doppler spectral data, wherein when all of the plurality of cardiac cycles are determined to be of low quality, the at least one processor (172, 126, 160, 136, 140) is further configured to determine whether individual ones of a plurality of cardiac cycles in the additional spectral Doppler data are of high quality or of low quality, based, at least in part on the heart rate variability, the acquisition quality, or the combination thereof.

6. The system (100) of claim 5, wherein when the buffer (142) does not include additional spectral Doppler data, the at least one processor (172, 126, 160, 136, 140) is configured to cause the display (138) to prompt a user to acquire the additional spectral Doppler data.

7. The system (100) of any one of claims 1 to 6, further comprising an ultrasound probe (112) configured to acquire the spectral Doppler data.

8. A method (900) comprising:

> determining (902) whether individual ones of a plurality of cardiac cycles in spectral Doppler data are of high quality or of low quality, based, at least in part on heart rate variability, acquisition quality, or a combination thereof; and
> displaying (904) the spectral Doppler data and a visual indication of one or more of cardiac cycles of the plurality of cardiac cycles determined to be of high quality and cardiac cycles of the plurality of cardiac cycles determined to be of low quality.

9. The method (900) of claim 8, further comprising:

> generating a spectrogram (200, 608, 708) based on the spectral Doppler data;
> extracting a spectral envelope (202, 610, 710) of the spectrogram; and
> determining the plurality of cardiac cycles within at least a portion of the spectral Doppler data based, at least in part, on the spectral envelope (202, 610, 710).

10. The method (900) of claim 9, wherein determining the plurality of cardiac cycles comprises:

> applying an auto-correlation function to the spectral envelope (202, 610, 710);
> determining an average cardiac cycle duration based, at least in part, on a first non-zero lag peak of the auto-correlation function; and
> locating, based at least in part, on the average cardiac cycle duration, a plurality of local maxima and a plurality of local minima in the portion of the spectral Doppler data, wherein individual ones of the plurality of cardiac cycles are located between individual ones of the plurality of local maxima and local minima.

11. The method (900) of any one of claims 8 to 10, wherein a cardiac cycle of the plurality of cardiac cycles is determined to be of low quality based on

> a comparison of the heart rate variability of the cardiac cycle to a threshold value, or
> a comparison of a plurality of cross-correlation coefficients for the cardiac cycle to a threshold value.

12. The method (900) of claim 11, wherein when a cardiac cycle of the plurality of cardiac cycles is determined to be of low quality based on a comparison of a plurality of cross-correlation coefficients for the cardiac cycle to a threshold value, the method further comprises:

calculating the plurality of cross-correlation coefficients by cross-correlating the cardiac cycle to other of the plurality of cardiac cycles.

13. The method (900) of any one of claims 10 to 12, further comprising determining (906) a parameter measurement based on cardiac cycles of the plurality of cardiac cycles determined to be high quality and optionally further comprising displaying (908) the parameter measurement.

14. The method (900) of claim 13, wherein the parameter comprises a resistive index, a peak systolic velocity (304), an end diastolic velocity (306), or a combination thereof.

15. A computer program product with instructions that when executed by at least one processor of a system, cause the system to perform the method of any one of claims 8 to 14.

FIG. 1

FIG. 2A

FIG. 2B

FIG. 3A

FIG. 3B

EP 4 331 500 A1

FIG. 4

FIG. 5B

FIG. 5A

FIG. 6

700

FIG. 7

FIG. 8

900

902

Determining whether
individual ones of a
plurality of cardiac
cycles in spectral
Doppler data are high
quality or low quality

904

Displaying the
spectral Doppler data
and a visual indication
of cardiac cycles of
the plurality of
cardiac cycles
determined to be
high quality and
cardiac cycles of the
plurality of cardiac
cycles determined to
be low quality

906

Determining a
parameter
measurement based
on cardiac cycles of
the plurality of
cardiac cycles
determined to be
high quality

908

Displaying the
parameter
measurement

FIG. 9

FIG. 10

Europäisches
Patentamt

European
Patent Office

Office européen
des brevets

# EUROPEAN SEARCH REPORT

Application Number

EP 22 19 8936

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2020/022676 A1 (HENDRIKS CORNELIS PETRUS [NL] ET AL) 23 January 2020 (2020-01-23) | 1-9,15 | INV. A61B8/08 A61B8/02 |
| Y | * abstract * <br> * figures 1-12 * <br> * paragraph [0007] – paragraph [0133] * <br> ----- | 10-14 | A61B8/00 |
| X | LIU GARRY ET AL: "Ultrasound-guided identification of cardiac imaging windows", MEDICAL PHYSICS, AIP, MELVILLE, NY, US, vol. 39, no. 6, 2 June 2012 (2012-06-02), pages 3009-3018, XP012161059, ISSN: 0094-2405, DOI: 10.1118/1.4711757 [retrieved on 2012-05-10] * abstract * <br> * figures 1-6 * <br> * Sections I – IV * <br> ----- | 1,8,15 | |
| Y | WO 2021/191092 A1 (KONINKLIJKE PHILIPS NV [NL]) 30 September 2021 (2021-09-30) * abstract * <br> * figures 1-8 * <br> * paragraph [0018] – paragraph [0085] * <br> ----- | 10-14 | |

TECHNICAL FIELDS SEARCHED (IPC)

A61B

The present search report has been drawn up for all claims

| Place of search | Date of completion of the search | Examiner |
|---|---|---|
| Munich | 24 December 2022 | Moehrs, Sascha |

CATEGORY OF CITED DOCUMENTS

X : particularly relevant if taken alone
Y : particularly relevant if combined with another
    document of the same category
A : technological background
O : non-written disclosure
P : intermediate document

T : theory or principle underlying the invention
E : earlier patent document, but published on, or
    after the filing date
D : document cited in the application
L : document cited for other reasons

& : member of the same patent family, corresponding
    document

EPO FORM 1503 03.82 (P04C01)

## ANNEX TO THE EUROPEAN SEARCH REPORT
## ON EUROPEAN PATENT APPLICATION NO.

EP 22 19 8936

24-12-2022

| Patent document cited in search report | | Publication date | Patent family member(s) | | Publication date |
|---|---|---|---|---|---|
| US 2020022676 | A1 | 23-01-2020 | EP | 3363369 A1 | 22-08-2018 |
| | | | EP | 3554385 A1 | 23-10-2019 |
| | | | JP | 6825106 B2 | 03-02-2021 |
| | | | JP | 2020512046 A | 23-04-2020 |
| | | | US | 2020022676 A1 | 23-01-2020 |
| | | | WO | 2018109052 A1 | 21-06-2018 |
| WO 2021191092 | A1 | 30-09-2021 | CN | 115334975 A | 11-11-2022 |
| | | | WO | 2021191092 A1 | 30-09-2021 |

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 6443896 B, Detmer

- US 6530885 B, Entrekin